# EUROPEAN PATENT SPECIFICATION

(11) **EP 1 701 613 B1**
(45) Date of publication and mention of the grant of the patent: **23.01.2008**
(21) Application number: 03782765.6
(22) Date of filing: 29.12.2003
(51) Int. Cl.: A01K 67/033, G01N 33/50

(54) **A PROCEDURE FOR SCREENING OF NEUROACTIVE SUBSTANCE AND THE ASSOCIATED NEURAL PLASTICITY**
EIN VERFAHREN ZUM SCREENING VON NEUROAKTIVEN SUBSTANZEN UND DIE ASSOZIERTE NEURALE PLASTIZITÄT
PROCEDE PERMETTANT DE CRIBLER UNE SUBSTANCE NEURO-ACTIVE ET LA PLASTICITE NEURONALE ASSOCIEE

(43) Date of publication of application: 20.09.2006
(73) Proprietor: COUNCIL OF SCIENTIFIC AND INDUSTRIAL RESEARCH, New Delhi 110 001 (IN)
(72) Inventor: SHARMA, Abhay, Inst. of Genomics & Integrative Bio, 110 007 Delhi (IN)
(74) Representative: van Westenbrugge, Andries
(86) International application number: PCT/IN2003/000402
(87) International publication number: WO 2005/063009

(56) References cited:
- US-A- 5 711 932
- US-B1- 6 291 739
- US-B2- 6 541 193

## Description

### FIELD OF THE INVENTION

The present invention relates to a method for the screening of neuroactive substance and the associated neural plasticity using an animal model. More particularly, the present invention relates to a method for the screening of neuroactive compounds using locomotor performance of the fruit fly *Drosophila melanogaster.* Further, by identifying in the fruit fly a behavioral characteristic which once affected by neuroactive drugs remain altered throughout the life even after the drugs are withdrawn, it presents a neural plasticity model with a potential to be used as a method to identify candidate disease genes and drug targets for neurological and psychiatric disorders.

### BACKGROUND AND PRIOR ART

By studying the effect of structurally and functionally diverse neuroactive compounds on locomotor activities in *Drosophila,* it is demonstrated that fruit fly could serve as a simple, rapid and inexpensive whole organism *in vivo* phenotype-based model for screening of drugs, compounds, natural products etc. for neuroactivity. The development of an animal model and the associated process for neuroactive drug screening is of immense value in the development and identification of drugs and their potential for treatment of neurological and neuropsychiatric disorders such as epilepsy, ataxia, Parkinson's disease, Huntington's disease and schizophrenia. In addition, it is demonstrated that drug induced alteration in climbing speed is always long-lasting. This locomotor effect of neuroactive drugs as class can be put to use for identifying genes or proteins in the pathway involved in neural plasticity. In turn, candidate disease genes and molecular targets for drug development may be identified. For example, it may potentially be used for identification of candidate genes for epileptogenesis and of candidate molecular targets for developing anti-epileptogenic therapy as well as adjunctive agents in the treatment of a variety of neuropsychaitric diseases associated with plasticity impairements.

Neuroactive drugs are used in the treatment of neurological and mental illnesses such as epilepsy.and schizophrenia. Although a large number of such drugs exist, there is a need to develop newer drugs for various reasons. For example, in many epileptic patients, seizures can be controlled with the established antiepileptic drugs (AEDs) such as
phenobarbital, phenytoin, carbamazepine, and valproate (Brodie and Dichter, 1996, N. Eng. J. Med 334: 168-175; Marson and Chadwick, 1996, Curr. Op. Neurol 9:103-106). However, around 25-30 % of patients continue to have seizures despite optimal therapy and others have unacceptable side effects (Brodie and Dichter, 1996, N. Eng. J. Med 334: 168-175). In recent years, a number of new AEDs such as gabapentin, lamotrigine, felbamate and clobazam have been developed (Macdonald and Grenfield, 1997, Curr. Op. Neurobiol. 10: 121-128). Adequate data on the possible adverse effects of these AEDs, however, is not available (Yerby, 2003, Epilepsia 44 Suppl 3: 33-40; Lathers et al, 2003, J. Clin. Pharmacol. 43: 491-503). In addition, these new drugs have limited efficacy and have the potential for serious side effects (Kwan and Brodie, 2003, Neurology 60: S2-S12). Clinical trials have shown that some patients respond to one drug in a better way than to another, even when they have similar types of seizures and the drugs used have similar mechanisms of action; the frequency and severity of side effects also vary substantially. In view of the above, it is clear that there is a need to develop more AEDs Schmidt, 2002, Epilepsy Res. 50: 21-32). Similarly, though newer antipsychotic drugs to treat neuropsychiatric disorders such as schizophrenia have been developed, there still exists a need to develop more (Goldstein, 1999, Drugs Today 35: 193-210; Sawa and Snyder, 2003, Expert. Opin. Investig. Drugs 11: 1335-1341; Scatton and Sanger, 2000, Pharmacol. 11: 243-256). Primary screening of random samples of natural products or combinatorial libraries for broad-based neuroactivity would be key to identification of potential compounds for further characterization of specific central nervous system (CNS) activity. *In silico* prediction, culture neuronal networks etc. are methods directed towards identification of CNS active compounds (Pancrazio et al, 2003, Biosens. Bioelectron 18: 39-47).

*D. melanogasten* has lately emerged as an attractive model to study drug induced behavior and addiction (Bellen, 1998, Cell 93: 909; Andretic et al, 1999, Science 285: 1066; Wolf and Heberlein, 2003, J. Neurobiol. 54: 161). Like mammals, dopaminergic pathways in *Drosophila* play a role in modulating locomotor behavior in response to neuroactive drugs (Bainton et al, 2000, Curr. Biol. 10: 187). Acute exposure to neuroactive compounds such as ethanol, nicotine and cocaine are known to affect locomotor behavior in both *Drosophila* as well as mammalian models (Heberlein, 2003, J. Neurobiol. 54: 161; Miller et al, 2001, Psychopharmacol. 56: 469; Bevins and Basheer, 2001, Physiol. Behav. 72: 237). It is important to note here that many therapeutic drugs are known to exhibit their CNS effect only after certain period of time.

*Drosophila melanogaster* ion channel mutants have earlier been validated as *in vivo* model for screening of antiepileptic and analeptic substance (Sharma and Kumar, US patent number 6291739; Sharma and Kumar, US patent number 6541193; Sharma *et al.,* US patent number 6617491; Kuebler and Tanouye, 2002, Brain Res. 958: 36-42). In the present invention, various locomotor activities in wild type fruitfly has been analysed secondary to chronic exposure to a wide variety, structurally and functionally, of CNS active drugs to develop a screening model. Six drugs - the four convulsants strychnine, pentylenetetrazol, pilocarpine-hydrochloride, and tetraethylammonium chloride, the mood stabilizer lithium carbonate, and the antiepileptic ethosuximide - have been used to determine if there is any specific locomotor activity that is affected by all the drugs individually. The drugs used are structurally different and act through a diversity of modes, as glycine-gated chloride channel antagonist, gamma-aminobutyric acid antagonist, muscarinic acetylcholine receptor agonist, K⁺ channel blocker, dopamine D2 receptor modulator, and T-type Ca⁺⁺ channel blocker, respectively.

Chronic exposure to neuroactive drugs including those of human use and abuse produces long lasting changes in neuronal function and behavior (1-3). Neural plasticity underlies these changes (3,4). Most important, involvement of regulation of gene expression and chromatin structure has recently been demonstrated in synaptic plasticity (5). *D*. *melanogaster* has lately emerged as an attractive model to study drug induced behavior and addiction (6,7). Exposure to various neuroactive compounds is known to affect locomotor behavior in both *Drosophila* as well as mammalian models (7-9). Like mammals, dopaminergic pathways in *Drosophila* play a role in modulating locomotor behavior in response to neuroactive drugs (10).

Dopamine is known to play a role in cognitive functions and behavior, influencing learning, memory, intelligence as well as eating-, sleep- and sexual- behavior etc (11-15). Dopaminergic disturbances are also believed to underlie aggressive behavior and suicidal tendency (16,17). Role of dopamine in drug addiction is well established (18). A defective dopaminergic transmission has been implicated in various neurological and psychiatric disorders such as narcolepsy, schizophrenia, depression, and attention deficit hyperactivity disorder (19-21).

Of the present interest was to examine various locomotor activities of flies under chronic treatment of a diversity of neuroactive drugs to see if each and every drug causes alteration in a particular locomotor activity. Further, it was investigated if any particular activity remains altered for a long time after the drugs are withdrawan. Identification of such behavioral characteristic would be expected to yield a drug screening procedure as well as a neural plasticity model relevant to drug testing and disease and drug candidate identification.

### OBJECTS OF THE INVENTION

The main object of the invention is the development of an animal model for the identification of neuroactive agents and the associated neural plasticity.

It is a further object of the invention to develop a method for the screening of neuroactive compounds and the associated neural plasticity using the fruit fly *Drosophila melanogaster.*

It is another object of the invention to develop a simple, inexpensive and rapid method for the screening of neuroactive agents and the associated neural plasticity.

It is a further object of the invention to develop an ethical animal model for the screening of neuroactive compounds and the associated neural plasticity that is also rapid, simple and inexpensive.

### SUMMARY OF THE INVENTION

The invention relates to a procedure for screening of neuroactive substance and the associated neural plasticity by treating the fruitfly *Drosophila melanogaster* adult males with fly medium containing either of the neuroactive compounds strychnine, pentylenetetrazol, pilocarpine hydrochloride, tetraethylammonium chloride, lithium carbonate and ethosuximide, subjecting flies to negative geotaxis and horizontal locomotor assays, observing the height climbed and distance walked by and the climbing speed of the flies wherein an altered height climbed by flies under drug treatment and a long-lasting alteration in climbing speed of flies after drug withdrawal is most characteristic of the neuractive compounds.

### DETAILED DESCRIPTION OF THE INVENTION

Accordingly, present invention relates to a rapid procedure for the screening of neuroactive substance, the said method comprising
(a) culturing *Drosophila melanogaster,*
(b) collecting 2-4 days old flies,
(c) separating males from females under ether anesthesia,
(d) treating the males of step (c) in the presence or absence of neuroactive drugs in the medium,
(e) subjecting the flies of step (d) to negative geotaxis and horizontal locomotor assays,
(f) examining locomotor activities of flies of step (e) in terms of height climbed, climbing speed and distance walked, wherein an alteration in either of the three locomotor activities in drug treated males, compared to those of normally fed flies, is characteristic of neuroactive compounds,
(g) withdrawing drugs from the diet of drug treated flies of step (d),
(h) subjecting the flies of step (g) to negative geotaxis and horizontal locomotion assays, and
(i) examining locomotor activity of flies of step (h) in terms of height climbed, climbing speed and distance walked, wherein an alteration in either of the three locomotor activities in drug treated males after drug withdrawal, compared to that of normally fed flies, is indicative of neural plasticity induced by neuroactive compounds.

In one embodiment of the invention, the locomotor activity identified as most characteristic of neuroactive compounds and the associated neural plasticity is selected from height climbed, climbing speed and distance walked.

In another embodiment of the invention, the neuroactive drugs used are selected from a group consisting of strychnine, pentylenetetrazol, pilocarpine hydrochloride, tetraethylammonium chloride, lithium carbonate and ethosuximide.

Still in one another embodiment of the present invention, it provides a simple, inexpensive and rapid method of using the fruit fly *Drosophila melanogaster* as a biological screen for neuroactive compounds and the associated neural plasticity wherein the agents to be screened could be fed to male flies followed by observing an altered locomotor activity that is characteristic of a neuroactive agent or the neural plasticity induced by it.

### BRIEF DESCRIPTION OF THE TABLES

- **Table 1**.: Height climbed, climbing speed and distance walked in cm, cm/sec and cm marks crossed per minute, respectively
- **Table 2**: Height climbed by flies in cm
- **Table 3**: Climbing speed of flies in cm/sec
- **Table 4**: Distance walked by flies in cm marks crossed per minute

### EXAMPLES

The invention is illustrated by the following examples, which are provided to illustrate the invention and should not be construed as limitation in the inventive concept herein.

### EXAMPLE 1

Unless otherwise mentioned, standard methods of fly manipulation were followed. Standard fly medium consisting of agar-agar, maize powder, brown sugar, dried yeast and nipagin was used. Flies were cultured at 22 ± 1°C, 60% RH, and 12 hrs light (9 AM to 9 PM) and 12 hours dark cycle. *D. melanogaster* wild type Oregon-R strain was used in the experiment. To obtain males for control and drug treatment, flies from identical cultures grown in glass vials were first allowed to lay eggs in milk bottles containing medium. Flies were shifted to fresh bottles every 12 hr. First 4 sets of bottles were discarded. Flies that emerged in subsequent bottles were only used. Those that emerged in the beginning were first discarded and then flies were collected twice at 12 hr interval. Flies collected each time were kept separately in a single bottle. Two days after first collection, males and females from both the bottles were separated. Males were then pooled together and shifted to a new bottle. Flies were used for control or drug treatment two days later.

### EXAMPLE 2

Following morning after pooling males for treatment, drugs (Sigma-Aldrich Co, St. Louis, U. S. A.) were first dissolved in distilled water at following concentration: 33.3 mg/ml of strychnine (STR), 40 mg/ml of pentylenetetrazol (PTZ), 20 mg/ml of pilocarpine-hydrochloride (PILO), 20 mg/ml of tetraethylammonium chloride (TEA), 10 mg/ml of lithium chloride (LICA), and 10.5 mg/ml of ethosuximide (ESD). Appropriate volume of freshly made drug solutions were then poured in molten fly media, and mixed thoroughly, to achieve a final concentration of 3.33 mg/ml of STR, 4 mg/ml of PTZ, 2 mg/ml of PILO, 2 mg/ml of TEA, 1 mg/ml of LICA, and 1.05 mg/ml of ESD. For control, i.e., normal food (NF), distilled water of same volume as drug solution was added in the medium and mixed. Following this, the molten media was dispensed in glass vials, stored overnight at 4°C and then used for above treatment of flies. Thirty male flies were shifted to each of the seven treatment vials, NF, STR, PTZ, PILO, TEA, LICA and ESD. Flies were maintained at 22 ± 1°C, 60% RH, and 12 hrs light (9 AM to 9 PM) and 12 hours dark cycle.

### EXAMPLE 3

Routine examination of gross locomotor behavior was carried out were at room temperature between 9 AM to 9 PM using startle induced group climbing test, by simultaneously tapping two treatment vials, one containing the control flies and the other, flies treated with either drug. The vials were tapped in inverted position, i.e., cotton side down, on a piece of packing foam so that all the flies are brought down at the bottom of the vial. Flies were then allowed to climb in inverted vials, standing undisturbed on the surface of the table. Climbing activities in the two vials were then visually compared to subjectively assess if there is any difference between the control and the drug groups. This exercise was repeated several times each in many sessions each day to arrive at either of the three possible alternatives - the drug groups climb faster than that of control, slower than that of control, or climb with a speed similar to that of control. During routine startle induced group climbing test, all the vials of a parallel set were similarly treated for same number of times. Drug vials were coded to eliminate any bias. In case of any doubt, several pairs of flies, a control and a drug fly in each pair, were separately examined. Also, drug-drug comparisons were often made for the same reason.

### EXAMPLE 4

Negative geotaxis assays for measuring height climbed and climbing speed, and horizontal locomotion assay for measuring distance walked - were performed at room temperature, between 9 AM to 9 PM. They were all performed at a specified area in a room, where same light sources were always used. While measuring various locomotor activities, extreme care was taken to ensure that the room is quiet, and the table onto which assays were performed undisturbed and vibration free. Utmost care was taken to ensure identical handling of flies, in minutest details. For measuring height climbed, climbing speed and distance walked, a single fly was randomly selected at a time. This was achieved by first inverting flies from a given treatment vial into an empty vial and then continuously and gently shaking and inverting both the vials in such a way that a single fly is finally trapped in the empty vial. Once trapped, all the three locomotor activities were measured using the same fly, height climbed, climbing speed and distance walked, in that order. One fly each from NF, STR, PTZ, PILO, TEA, LICA, and ESD, selected randomly, was first scored and then the same exercise was repeated for another two flies. Once a fly was scored for all the three locomotor activities, it was discarded. Flies were particularly checked for intact legs and wings, before they were used in negative geotaxis and horizontal locomotion assays.

### EXAMPLE 5

A glass tube of 1.7 cm diameter and 30. or 36 mm length, along with two cotton plugs were used in negative geotaxis and horizontal locomotion assays. The tube was length wise marked with lines at every cm. Three flies from each treatment were assayed. Each fly was assayed 10 times in succession. Each fly was first familiarized in the tube by keeping it for a minute in vertically or horizontally placed tube, before negative geotaxis or horizontal locomotion assay, respectively was performed. Both the height climbed and the climbing speed was measured in the negative geotaxis assay. Horizontal locomotion assay was performed for measuring distance walked. In negative geotaxis assay, a single fly was trapped inside the tube. The fly was brought to the bottom of the tube, by tapping the tube on a piece of packing foam. As soon as the fly had fallen on the cotton plug at the bottom, the tube was as such placed vertically on the surface of the work table. The height climbed by the fly in cm and the time taken to climb in sec were both recorded. Climbing was considered complete when the fly either touched the cotton plug at the top, fell to the bottom after climbing a certain height, or stopped after climbing to a certain height for more than approximately 5 sec. While climbing, flies sometimes, rarely though, jumped and/or took flights upwards or downwards. Unless these activities looked unusual, they were accepted. Spiral movement, uncommon though, during climbing was accepted. Downward movement, rare though, during climbing was also accepted, unless it continued for an unusually long time. Climbing to a height less than 7 cm was not considered in the assay. In horizontal locomotion assay, a singly fly was first brought to the middle of the tube by gentle shaking and then the fly was constantly monitored to count how many lines it walked across in a minute. Any single jump, short or long, was counted as one. Usually flies walked straight along the upper surface towards one end of the tube, explored there by moving around the inner periphery for some time, and then moved towards the other end, and so on. Though uncommon, they also walked along the lower surface, moved in a spiral fashion, explored much at one end, and stopped at times for shorter or longer duration. All these variations were accepted in the assay.
Routine examination of control and drug treated flies for gross locomotor behavior in startle-induced group climbing test showed an altered locomotor activity in flies exposed to drugs. On day 7 of the beginning of treatment, individual flies were used in negative geotaxis and horizontal locomotion assays. Compared to NF, the control, STR, PTZ, PILO, TEA, LICA, and ESD caused an increase in the height climbed by drug exposed flies in negative geotaxis assay (Table 1).

**Table 1.**

| **Specific locomotor activity** | **NF** | **STR** | **PTZ** | **PILO** | **TEA** | **LICA** | **ESD** |
|---|---|---|---|---|---|---|---|
| *Height climbed* | | | | | | | |
| Mean | 20.96 | 33.36*** | 29.13*** | 34.66*** | 32*** | 35.8*** | 25.66* |
| SE | 1.52 | 0.92 | 1.69 | 0.75 | 1.33 | 0.2 | 1.66 |
| *Climbing speed* | | | | | | | |
| Mean | 1.02 | 1.53*** | 1.63*** | 1.52*** | 1.67*** | 1.9*** | 0.98 |
| SE | 0.04 | 0.09 | 0.07 | 0.07 | 0.1 | 0.08 | 0.03 |

| *Distance walked* | | | | | | | |
|---|---|---|---|---|---|---|---|
| Mean | 29.1 | 39.33* | 42.5** | 48.53*** | 45.1*** | 34.23 | 37.1 |
| SE | 2.7 | 3.8 | 3.52 | 2.78 | 2.44 | 3.05 | 4.16 |

Measurement of climbing speed in negative geotaxis assay showed an increase in case of all the drugs except ESD (Table 1). In horizontal locomotion assay, all the drugs except LICA and ESD caused an increase in the distance walked (Table 1). In brief, the results demonstrated that all the drugs alter height climbed by flies in the negative geotaxis assay. This alteration is therefore a feature common to neuroactive drugs as a class.

On 17^{th} day of the beginning of the treatment, the drugs were withdrawn by shifting all NF as well as drug exposed flies to NF media. On 21^{st} and 48^{th} day, of the beginning of the treatment, i.e., on 4^{th} and 31^{st} day of drug withdrawal, height climbed, climbing speed and distance walked was again measured. On 4^{th} day after drug withdrawal, only ESD of all the drugs showed an increase, compared to NF, in height climbed (Table 2).

**Table 2**

| | NF | STR | PTZ | PILO | TEA | LICA | ESD |
|---|---|---|---|---|---|---|---|
| 4^{th} day | | | | | | | |
| Mean | 23.2 | 26.26 | 20.3 | 19.96 | 22.13 | 25.33 | 32.66*** |
| SE | 1.61 | 1.75 | 1.71 | 1.89 | 1.82 | 1.77 | 1.2 |
| 31at day | | | | | | | |
| Mean | 13.66 | 15.8 | 9.93*** | 16.46 | 16.2 | 11.4* | 19.5*** |
| SE | 0.53 | 1 | 0.35 | 1.72 | 1.54 | 0.78 | 1.47 |

Days are after drug withdrawal. Number of observation *n* = 30. Student's t-test, unpaired, was applied to test the significance of difference between control and drug means. Astricks denote the level of significance; single, 5%; double, 1%; triple, 0.1%.

On 31^{st} day, flies exposed earlier to PTZ, and LICA exhibited a lower and ESD a greater climbing speed than the control (Table 2). In brief, a long-lasting change in height climbed is not produced by neuroactive drugs as a class. On 4^{th} day after drug withdrawal, all drugs except TEA showed an increase, compared to NF, in climbing speed (Table 3).

**Table 3**

| | NF | STR | PTZ | PILO | TEA | LICA | ESD |
|---|---|---|---|---|---|---|---|
| 4^{th} day | | | | | | | |
| Mean | 1.03 | 1.29** | 1.25** | 1.32** | 1.11 | 1.22* | 1.5*** |
| SE | 0.05 | 0.07 | 0.05 | 0.06 | 0.04 | 0.05 | 0.06 |
| 31 at day | | | | | | | |
| Mean | 0.88 | 1.04* | 1.37*** | 1.14** | .1.16*** | 1.3*** | 1.52*** |
| SE | 0.05 | 0.04 | 0.07 | 0.05 | 0.04 | 0.040.07 | |

Days are after drug withdrawal. Number of observation *n* = 30. Student's t-test, unpaired, was applied to test the significance of difference between control and drug means. Astricks denote the level of significance; single, 5%; double, 1%; triple, 0.1%.

On 31^{st} day, flies exposed earlier to either drug exhibited a higher climbing speed than the control (Table 3). In brief, a long-lasting change in climbing speed is produced by neuroactive drugs as a class. On 4^{th} day of the beginning of the treatment, all drugs except STR, ESD and NICO showed a decrease, compared to NF, in distance walked (Table 4).

**Table 4**

| | NF | STR | PTZ | PILO | TEA | LICA | ESD |
|---|---|---|---|---|---|---|---|
| 4^{th} day | | | | | | | |
| Mean | 48.26 | 43.66 | 28.03*** | 33.83** | 26.76*** | 33*** | 40.16*** |
| SE | 2.69 | 3.91 | 4.59 | 3.23 | 3.37 | 2.56 | 3.43 |
| 31 at day | | | | | | | |
| Mean | 24.23 | 27.66 | 12.5* | 21.16 | 27.16 | 6.93*** | 30.63 |
| SE | 4.17 | 3.75 | 2.1 | 4.97 | 5.28 | 1.44 | 4.1 |

Days are after drug withdrawal. Number of observation *n* = 30. Student's t-test, unpaired, was applied to test the significance of difference between control and drug means. Astricks denote the level of significance; single, 5%; double, 1%; triple, 0.1%.

On 31^{st} day of the beginning of the treatment, i.e., on 31^{st} day of drug withdrawal. On this day, flies exposed earlier to PTZ and LICA only exhibited a lesser distance walked than the control (Table 4). In brief, a long-lasting change in distance walked was not produced by neuroactive drugs as a class.

It is important to note here that many drugs used in the present invention are known to cause movement disorders in man (Blanchet, 2003, Can. J. Neurol. Sci. 30S1, S101). Like mammals, dopaminergic pathways in *Drosophila* play a role in modulating locomotor behavior in response to neuroactive drugs (Bainton et al, 2000, Curr. Biol. 10: 187). The present results therefore show that these drugs in general are capable of affecting dopaminergic system. This highlights the integrative characteristics of the nervous system. Dopamine is known to play a role in cognitive functions and behavior, influencing learning, memory, intelligence as well as eating- , sleep- and sexual- behavior etc. (Mozley et al, 2001, Am. J. Psychiatry 158, 1492; Setlow and McGaugh, 2000, Learn. Mem. 7, 187; Tsai et al, 2002, Neuropsychopharmacology 45, 128; Bailer and Kaye, 2003, Curr. Drug Target CNS Neurol. Disord. 2, 53; Saint et al, 2000, Neuroreport 11, 1619). Dopaminergic disturbances are also believed to underlie aggressive behavior and suicidal tendency (Miczek et al, 2002, Psychopharmacology 163, 438; Pitchot et al, 2001, Eur. Psychiatry 16, 424). Role of dopamine in drug addiction is well established (Phillips et al, 2003, Nature 422, 614). A defective dopaminergic transmission has been implicated in various neurological and psychiatric disorders such as narcolepsy, schizophrenia, depression, and attention deficit hyperactivity disorder (Eisensehr et al, 2003, Neurology 60, 1817; Davids et al, 2003, Brain Res. Brain res. Rev. 42, 1; Baumeister and Francis, 2002, J. Hist. Neurosci. 11, 265).

Effect of drugs such as ethanol vapor, volatilized cocaine and volatilized nicotine on locomotor behavior in *Drosophila* has been described earlier (22). However, the drug exposure time, dosage and route of administration applied in these studies were basically designed for acute drug treatment. The present procedure identifies locomotor activity changes associated with chronic exposure to drugs. This is important because many neuroactive drugs are known to produce their therapeutic effects long after beginning of treatment. It is important to note here that quantification of ethanol-induced changes in walking activity using simple line crossing assays has been found to be essentially identical to that achieved using automated inebriometer assays (22).

Mechanisms underlying drug-induced neural plasticity is considered to represent general ways of neural systems adapting to physiological and behavioral stimuli. Relevant prior art on drug model includes cocaine-induced locomotor activation in vertebrate and fruitfly models (23-25). Also, locomotor effects of ethanol and nicotine in *Drosophila* have been studied (22). In *Drosophila,* exposure to ethanol and cocaine cause development of tolerance and sensitivity, respectively (22). In ethanol tolerance, previously exposed flies, compared to drug naïve individuals, require higher ethanol doses to elicit a particular behavioral response. In cocaine sensitization, a smaller dose is required instead. The differential response of previously exposed versus naïve flies have been observed in experiments where the second dose was administered after an interval of 4-6 hrs (22). In the current paradigm, drug induced alteration in locomotor activity has been found to last for weeks, till it was examined. The present neural plasticity model is however novel because, unlike the prior art, it is a chronic drug use induced neural plasticity model where the drug induced behavioral alteration persists throughout the life of the organism. It is important because it is long-term plastic changes in brain function that may be more relevant to drug therapy and diseases.

### ADVANTAGES OF THE INVENTION

1. The procedure described here is simple, inexpensive and rapid, ideal for primary screening of CNS active substance as well as for preparing brain samples in batches for molecular analysis towards drug target identification.
2. The neural plasticity model presented is applicable for investigating both drug specific as well as core processes involved in producing long-lasting changes.
3. The method is non-invasive and therefore more suitable to minimize occurrence of false positives and false negatives that might arise as experimental artifacts in the screening.
4. Using *Drosophila,* an invertebrate, for drug research is not unethical from point of view of animal rights.

### References:

1. Brent L, Chandler P, Fierz W, Medawar PB, Rayfield LS, Simpson E. Further studies on supposed lamarckian inheritance of immunological tolerance. Nature 1982; 295:242-44.McLaren A, Chandler P, Buehr M, Fierz W, Simpson E. Immune reactivity of progeny of tetraparental male mice. Nature 1981; 290:513-14.
2. Cavalli G, Paro R. Epigenetic inheritance of active chromatin after removal of the main transactivator. Science 1999; 286:955-58.
3. Coyle JT, Duman RS. Finding the intracellular signaling pathways affected by mood disorder treatments. Neuron 2003; 38:157-160.
4. Darboux I, Lingueglia E, Champigny G, Coscoy S, Barbry P, Lazdunski M. dGNaC1, a gonad-specific amiloride-sensitive Na+ channel. J Biol Chem 1998; 273:9424-29.
5. Davids E, Zhang K, Tarazi FI, Baldessarini RJ. Animal models of attention-deficit hyperactivity disorder. Brain Res Brain Res Rev 2003; 42:1-21.
6. de Saint Hilaire Z, Orosco M, Rouch C, Python A, Nicolaidis S. Neuromodulation of the prefrontal cortex during sleep: a microdialysis study in rats. Neuroreport 2000; 11:1619-24.
7. De Sarro A., Naccari F, De Sarro G. Enhanced susceptibility of pentylenetetrazole kindled mice to quinolone effects. Int J Antimicrob Agents 1999; 12:239-244.
8. Dennis C. Altered states. Nature 2003; 421:686-88.
9. Eisensehr I, Linke R, Tatsch K, Von Lindeiner H, Kharraz B, Gildehaus FJ, Eberle R, Pollmacher T; Schuld A, Noachtar S. Alteration of the striatal dopaminergic system in human narcolepsy. Neurology 2003; 60:1817-19.
10. Eisses KT. Concurrent teratogenic and mutagenic action of 2-methoxyethanol in Drosophila melanogaster larvae resulted in similar phenotypes: close resemblance to directed mutations. Teratog Carcinog Mutagen 1999; 19:183-204.
11. Mozley LH, Gur RC, Mozley PD, Gur RE. Striatal dopamine transporters and cognitive functioning in healthy men- and women. Am J Psychiatry 2001; 158:1492-99.
12. Setlow B, McGaugh JL. D2 dopamine receptor blockade immediately post-training enhances retention in hidden and visible platform versions of the water maze. Learn Mem 2000; 7:187-191.
13. Tsai SJ, Yu YW, Lin CH, Chen TJ, Chen SP, Hong CJ. Dopamine D2 receptor and N-methyl-D-aspartate receptor 2B subunit genetic variants and intelligence. Neuropsychobiology 2002; 45:128-30.
14. Bailer UF, Kaye WH. A review of neuropeptide and neuroendocrine dysregulation in anorexia and bulimia nervosa. Curr Drug Target CNS Neurol Disod 2003: 2:53-59.
15. de Saint Hilaire Z, Orosco M, Rouch C, Python A, Nicolaidis S. Neuromodulation of the prefrontal cortex during sleep: a microdialysis study in rats. Neuroreport 2000; 11:1619-24.
16. Miczek KA, Fish EW, De Bold JF, De Almeida RM. Social and neural determinants of aggressive behavior: pharmacotherapeutic targets at serotonin, dopamine and gamma-aminobutyric acid systems. Psychopharmacology 2002; 163:438-58.
17. Pitchot W, Hansenne M, Ansseau M. Role of dopamine in non-depressed patients with a history of suicide attempts. Eur. Psychiatry 2001; 16:424-27.
18. Phillips PE, Stuber GD, Heien ML, Wightman RM, Carelli RM. Subsecond dopamine release promotes cocaine seeking. Nature 2003; 422:614-18.
19. Eisensehr I, Linke R, Tatsch K, Von Lindeiner H, Kharraz B, Gildehaus FJ, Eberle R, Pollmacher T, Schuld A, Noachtar S. Alteration of the striatal dopaminergic system in human narcolepsy. Neurology 2003; 60:1817-19.
20. Davids E, Zhang K, Tarazi FI, Baldessarini RJ. Animal models of attention-deficit hyperactivity disorder. Brain Res Brain Res Rev 2003; 42:1-21.
21. Baumeister AA, Francis JL. Historical development of the dopamine hypothesis of schizophrenia. J Hist Neurosci 2002; 11:265-77.
22. Wolf FW, Heberlein U. Invertebrate models of drug abuse. J Neurobiol. 2003; 54:161-78.
23. McClung C, Hirsh J. Stereotypic behavioral responses in free-base cocaine and the development of behavioral sensitization in Drosophila. Curr. Biol. 1998; 8:109-12.
24. Andretic R, Chaney S, Hirsh J. requirement of circadian genes for cocaine sensitization in Drosophila. Science 1999; 285:1066-8.
   Abarca C, Albrecht U, Spanagel R. Cocaine sensitization and reward are under the influence of circadian genes and rhythm. Proc. Natl. Acad. Sci. USA 2002; 99:9026-30.

## Claims

1. A rapid process for screening of neuroactive substance and the associated neural plasticity in a subject wherein said process comprising the steps:
(a) culturing *Drosophila melanogaster;*
(b) collecting flies of a single age group;
(c) separating males from females flies of step (b);
(d) treating the males of step (c) in the presence or absence of neuroactive drugs in the medium comprising agar-agar, maize powder, brown sugar, dried yeast and nipagin;
(e) subjecting the flies of step (d) to negative geotaxis and horizontal locomotor assays;
(f) examining locomotor activities of flies of step (e) in terms of height climbed, climbing speed and distance walked, wherein an alteration in either of the three locomotor activities in drug treated males, compared to those of normally fed flies, is characteristic of neuroactive compounds;
(g) withdrawing drugs from the diet of drug treated flies of step (d);
(h) subjecting the flies of step (g) to negative geotaxis and horizontal locomotion assays, and
(i) examining locomotor activity of flies of step (h) in terms of height climbed, climbing speed and distance walked, wherein an alteration in either of the three locomotor activities in drug treated males after drug withdrawal, compared to that of normally fed flies, is indicative of neural plasticity induced by neuroactive compounds.

2. A process as claimed in claim 1, wherein the subject is *Drosophila melanogaster.*

3. A process as claimed in claim 1, wherein in step (b) the age is in the range of 2 to 4 days.

4. The method of claim 1, wherein an altered height climbed by flies under drug treatment and an altered climbing speed of flies after drug withdrawal are selected as most characteristic of neuroactive compounds.

## Patentansprüche

1. Schnelles Verfahren zum Screening von neuroaktiven Substanzen und die damit verbundene neurale Plastizität in einem Subjekt, wobei das Verfahren die folgenden Schritte umfasst:
(a) Züchten von Drosophila melanogaster,
(b) Sammeln von Fliegen einer bestimmten Altersgruppe,
(c) Trennen der männlichen von den weiblichen Fliegen aus Schritt (b),
(d) Behandeln der Männchen aus Schritt (c) durch Vorhandensein oder Fehlen von neuroaktiven Arzneimitteln in dem Medium, umfassend Agar-Agar, Maispulver, braunen Zucker, getrocknete Hefe und Nipagin,
(e) Unterziehen der Fliegen aus Schritt (d) Untersuchungen zur negativen Geotaxis und horizontalen Bewegung,
(f) Untersuchen der Bewegungsaktivitäten der Fliegen aus Schritt (e) hinsichtlich aufgestiegener Höhe, Aufstiegsgeschwindigkeit und gelaufener Entfernung, wobei eine Änderung in einer der drei Bewegungsaktivitäten bei medikamentös behandelten Männchen, im Vergleich zu denen normal ernährter Fliegen, für neuroaktive Verbindungen charakteristisch ist,
(g) Herausnehmen der Arzneimittel aus der Diät der medikamentös behandelten Fliegen aus Schritt (d),
(h) Unterziehen der Fliegen aus Schritt (g) Untersuchungen zur negativen Geotaxis und horizontalen Fortbewegung, und
(i) Untersuchen der Bewegungsaktivität der Fliegen aus Schritt (h) hinsichtlich aufgestiegener Höhe, Aufstiegsgeschwindigkeit und gelaufener Entfernung, wobei eine Änderung in einer der drei Bewegungsaktivitäten bei medikamentös behandelten Männchen nach Arzneimittelentzug, im Vergleich zu denen normal ernährter Fliegen, bezeichnend ist für die neurale Plastizität, die durch neuroaktive Verbindungen eingeleitet wird.

2. Verfahren nach Anspruch 1, wobei das Subjekt eine Drosophila melanogaster ist.

3. Verfahren nach Anspruch 1, wobei in Schritt (b) das Alter im Bereich von 2 bis 4 Tagen liegt.

4. Methode nach Anspruch 1, wobei eine geänderte Höhe, die die Fliegen unter medikamentöser Behandlung aufgestiegen sind, und eine geänderte Aufstiegsgeschwindigkeit der Fliegen nach Arzneimittelentzug als am charakteristischsten für neuroaktive Verbindungen ausgewählt sind.

## Revendications

1. Procédé rapide pour le criblage d'une substance neuroactive et de la plasticité neuronale associée chez un sujet dans lequel ledit procédé comprend les étapes de :
(a) cultiver *Drosophila melanogaster*
(b) rassembler les mouches d'un groupe d'âge unique;
(c) séparer les mouches mâles des femelles de l'étape (b) ;
(d) traiter les mâles de l'étape (c) en présence ou en absence de médicaments neuroactifs dans le milieu comprenant de l'agar -agar, de la poudre de maïs, du sucre brun, de la levure séchée et de la nipagine ;
(e) soumettre les mouches de l'étape (d) à des tests de géotaxie négative et de locomotion horizontale ;
(f) examiner les activités locomotrices des mouches de l'étape (e) en termes de hauteur grimpée, de vitesse de grimpée et d e la distance parcourue en marchant, où une modification d'une des trois activités locomotrices chez les mâles traités avec le médicament, par rapport à celles de mouches nourries normalement, est caractéristique des composés neuroactifs ;
(g) retirer les médicaments du régime alimentaire des mouches traitées avec le médicament de l'étape (d) ;
(h) soumettre les mouches de l'étape (g) aux tests de géotaxie négative et de locomotion horizontale, et
(i) examiner l'activité locomotrice des mouches de l'étape (h) en termes de hauteur grimpée, de vitesse de grimpée et de distance parcourue en marchant, où une modification de l'une des trois activités locomotrices chez les mâles traités avec le médicament après le retrait du médicament, par rapport à celle des mouches alimentées normalement, est un indicateur de la plasticité neuronale induite par les composés neuroactifs.

2. Procédé selon la revendication 1, dans lequel le sujet est *Drosophila melanogaster.*

3. Procédé selon la revendication 1, dans lequel dans l'étape (b) l'âge se situe dans la plage de 2 à 4 jours.

4. Procédé selon la revendication 1, dans lequel une hauteur grimpée modifiée par des mouches sous traitement médicamenteux et une vitesse de grimpée modifiée des mouches après le retrait du médicament sont choi sies comme les plus caractéristiques des composés neuroactifs.
